# EUROPÄISCHE PATENTSCHRIFT

(11) **EP 3 100 748 B1**
(45) Veröffentlichungstag und Bekanntmachung des Hinweises auf die Patenterteilung: **13.06.2018**
(21) Anmeldenummer: 16165403.3
(22) Anmeldetag: 14.04.2016
(51) Int. Cl.: A61L 15/22, A61L 15/56, C12Q 1/04, A61F 13/84

(54) **WUNDAUFLAGE**
WOUND DRESSING
PANSEMENT

(43) Veröffentlichungstag der Anmeldung: 07.12.2016
(73) Patentinhaber: Sefar AG, 9410 Heiden (CH)
(72) Erfinder: GERDES, Gerd, 9030 Abtwil (CH); WEBER, Jérémie, 6833 Klaus (AT)
(74) Vertreter: Wunderlich & Heim Patentanwälte Partnerschaftsgesellschaft mbB

(56) Entgegenhaltungen:
- EP-A1- 2 153 807
- WO-A1-2012/171922

## Beschreibung

Die Erfindung betrifft eine Wundauflage mit einem Markermaterial zum Anzeigen eines Zustands einer Wunde, insbesondere eines mikrobiologischen Wundmilieus, gemäß dem Oberbegriff des Anspruchs 1.
Die Erfindung betrifft weiterhin ein Verfahren zum Herstellen einer Wundauflage mit einem Markermaterial gemäß dem Anspruch 15.
Aus der WO 2000/024438 A1 ist eine Zusammensetzung bekannt, welche eine mikrobiologische Verkeimung an medizinischen Geräten und Materialien anzeigen kann. Dieser Indikator umfasst einen Stoff oder eine Stoffzusammensetzung, welche abhängig von einem Vorhandensein eines mikrobiologischen Milieus zu einem Farbumschlag führen kann. Hierdurch wird medizinischem Personal signalisiert, dass eine Verkeimung vorhanden ist oder zumindest ein bestimmtes Niveau erreicht hat.

Die WO2012/171922 A1 offenbart eine Wundauflage mit einem Indikator zur Feststellung von mikrobieller Kontamination einer Wunde, umfassend eine wundzugewandte Signalschicht und eine Deckschicht.

Weiterhin ist es bekannt, derartige Indikatorstoffe auch bei Wundauflagen vorzusehen. Hierdurch kann medizinischem Personal frühzeitig ein Hinweis zu einem Wundverlauf gegeben werden. Insbesondere kann durch einen entsprechenden Indikator angezeigt werden, dass ein Wechsel der Wundauflage aufgrund des Erreichens eines bestimmten Verkeimungsniveaus sinnvoll oder notwendig ist.
Es bestehen jedoch nicht unerhebliche Schwankungen beim Ansprechen des Indikatormaterials. Ein Grund hierfür kann darin gesehen werden, dass etwa ein Austreten von Exsudat oder Sekret aus der Wunde einen nicht unerheblichen Einfluss auf den Zeitpunkt der Anzeige eines Farbumschlages haben kann. Ist das Wundauflagematerial in unmittelbarem Kontakt mit der Wunde, kann eine frühzeitige Anzeige erfolgen. Hat die Wundauflage hingegen einen gewissen Abstand zum Gewebe der Wunde, kann die Anzeige einer Verkeimung möglicherweise erst verspätet erfolgen. Dies kann für den Wundheilungsprozess nachteilig sein.
Der Erfindung liegt die **Aufgabe** zugrunde, eine Wundauflage und ein Verfahren zum Herstellen der Wundauflage anzugeben, mit welchen ein Zustand der Wunde besonders zuverlässig angezeigt werden kann.
Die Aufgabe wird zum einen durch eine Wundauflage mit den Merkmalen des Anspruchs 1 und zum anderen durch ein Verfahren mit den Merkmalen des Anspruchs 15 gelöst. Bevorzugte Ausführungsformen sind in den abhängigen Ansprüchen angegeben.
Die Erfindung ist dadurch gekennzeichnet, dass die Wundauflage eine Auflageseite aufweist, welche durch ein biokompatibles Monofilamentgewebe aus Monofilamentfäden mit einer glatten Oberfläche gebildet ist und dass auf dem Monofilamentgewebe an seiner von der Wunde abgewandten Oberseite mindestens ein Deckelement angebracht ist, welches mit Markermaterial versehen ist.
Ein Aspekt der Erfindung besteht darin, dass das Deckelement mit dem Markermaterial nicht unmittelbar mit dem verletzten Gewebe der Wunde in Kontakt steht. Vielmehr ist zwischen der Wunde und dem Deckelement mit dem Markermaterial eine Trennschicht vorgesehen, welche gemäß der Erfindung aus einem biokompatiblen Monofilamentgewebe gebildet ist. Das Monofilamentgewebe ist aus feinen drahtähnlichen Monofilamentgarnen gebildet. Im Vergleich zu Stapelfasern oder Multifilamentgarnen ist ein Eindringen von Wundsekret oder von Markermaterial in die Monofilamentgarne selbst und eine dadurch bedingte verstärkte Verkeimung bei den Monofilamentgarnen strukturbedingt nicht möglich. Hierdurch kann einer vorzeitigen Anzeige eines noch nicht kritischen Verkeimungszustandes vorgebeugt werden. Gleichzeitig kann das Deckelement mit dem Markermaterial so ausgebildet werden, dass dieses erst bei einer bestimmten Verkeimung hinter dem Monofilamentgewebe als Trennschicht anspricht.
Zudem wird durch die Trennschicht des Monofilamentgewebes einer Irritation der Wunde durch das Deckelement und das darin vorgesehene Markermaterial entgegengewirkt. Derartige Wechselwirkungen können zu einer Störung der Wundheilung führen.

Gemäß einem weiteren Aspekt der Erfindung ist das Monofilamentgewebe biokompatibel ausgebildet. Die Auswahl der Rohstoffe und die Verarbeitung zu dem Monofilamentgewebe ist dabei so gestaltet, dass das Monofilamentgewebe nicht irritierend ist, insbesondere nicht zytotoxisch, pyrogenfrei und hypoallergen ist. Gewebe, wie sie üblicherweise in der Industrie eingesetzt sind, sind grundsätzlich nicht biokompatibel. Biokompatible Gewebe sind aber aus dem Bereich der Blutfiltration bekannt. Von einer Biokompatibilität des Gewebes kann insbesondere dann ausgegangen werden, wenn die Anforderungen nach dem relevanten Kapitel von ISO 10993 oder des USP Plastics Class VI Tests (United States Pharmacopeia) erfüllt sind.

Durch die Biokompatibilität und Struktur des Garnes wird erreicht, dass einerseits eine Irritation des verletzten Gewebemateriales durch das Wundauflagematerial weitgehend vermieden wird und andererseits beim Ablösen des Wundauflagemateriales die Gefahr einer Beeinträchtigung, insbesondere eines Aufreißens, der Wunde entgegengewirkt ist.

Darüber hinaus kann das biokompatible Monofilamentgewebe so stabil ausgebildet, dass es als eine Trägerschicht dient, auf welcher mindestens ein Deckelement fest angebracht ist. Dieses Deckelement kann dabei selbst eine einzelne Schicht oder mehrere Schichten aufweisen. Entsprechend dem Einsatzzweck der Wundauflage kann das Deckelement zusätzlich in einer gewünschten Weise, etwa zum Aufnehmen von Wundsekret, oder zum Aufbringen von wundheilungsfördernden Mitteln, etwa einer Salbe oder einer Flüssigkeit, ausgebildet sein.

Gemäß der Erfindung können verschiedene Elemente und Materialien zum Bilden des Deckelementes vorgesehen sein, da diese auf der von der Auflageseite des Monofilamentgewebes abgewandten Oberseite angeordnet sind und somit nicht unmittelbar mit dem Gewebematerial im Wundbereich in Kontakt gelangen. Das Deckelement kann selbst einen mehrschichtigen Aufbau aufweisen.

Eine bevorzugte Ausführungsform der Erfindung besteht darin, dass das Monofilamentgewebe als ein Einfachgewebe mit offenmaschiger Struktur ausgebildet ist, welches eine Porengröße von 5 µm bis 500 µm, vorzugsweise 20 µm bis 300 µm, aufweist, und dass die Poren einen Anteil von 15% bis 70% der Fläche des Monofilamentgewebes bilden. Das erfindungsgemäße Monofilamentgewebe ist gemäß dieser Ausführungsform also besonders feinporig, so dass zwar einerseits ein Luft- und Flüssigkeitsdurchtritt durch die Poren möglich ist, jedoch andererseits ein Verwachsen oder Umgreifen des Monofilamentgewebes durch Gewebematerial kaum möglich ist. Es wird so eine schützende, aber durchlässige Wundauflage geschaffen, welche jedoch weiterhin eine klare Abgrenzung und damit eine leichte Lösbarkeit von der Wunde sicherstellt. Die gute Durchlässigkeit für Luft und Flüssigkeit ist insbesondere dadurch gegeben, dass die Poren einen Anteil von 15% bis 70% der Fläche des Monofilamentgewebes, vorzugsweise 40% bis 70%, bilden. Auf diese Weise wird eine hohe Durchlässigkeit erreicht.

Dabei ist das Monofilamentgewebe als ein Einfachgewebe ausgebildet, welches eine im Wesentlichen zweidimensionale Gewebekonstruktion mit einem einzelnen Kettfadensystem und einem einzelnen Schussfadensystem aufweist. Durch die Verwendung eines transparenten Fadenmateriales für die Monofilamentfäden wird zudem in Kombination mit der großen Porenfläche eine sehr hohe Transparenz erreicht. Trotz einer relativ dünnen Gewebedicke kann eine ausreichende Stabilität der durch das Monofilamentgewebe gebildeten Trägerschicht bei einer guten Gewebeoffenheit durch eine Leinwandbindung ein Taffet 1:1 erreicht werden. Eine weitere stabile Konstruktion kann durch eine Köperbindung in beliebigen Rapporten, bevorzugt in einer Bindung 1:2, 1:3, 2:2 und 3:3 betreffend Kettfaden-/Schussfaden-Anordnung, erreicht werden. Eine weitere stabile Bindung ist durch eine Atlasbindung in beliebigen Rapporten möglich, besonders bevorzugt in einer Atlasbindung 1:7 betreffend Kettfaden-/Schussfaden-Anordnung. Eine weitere mögliche stabile Bindung für das Monofilamentgewebe ist eine Tressenbindung.

Gemäß einer Weiterbildung der Erfindung ist es bevorzugt, dass die Monofilamentfäden des Monofilamentgewebes aus PA, PET, PP, PVDF und/oder PTFE gebildet sind und eine Durchmessergröße zwischen 20 µm bis 500 µm, vorzugsweise zwischen 30 µm bis 150 µm, aufweisen. Die Monofilamentfäden sind dabei aus besonders reinem Ausgangsmaterial hergestellt, so dass diese lebensmittelkonform sind. Es können pyrogenfreie und nicht zytotoxische Gewebe hergestellt werden. Als ein besonders bevorzugtes Material ist Polyester in einem gereinigten und biokompatiblen Zustand. Die Fadendurchmesser können je nach Anwendungsfall variieren, sind jedoch vorzugsweise möglichst klein gehalten. Insbesondere sind die Monofilamentfäden mit einer Längsrippenstruktur ausgebildet, bei welchen die Längsrippen von einem Kerndurchmesser des Monofilamentfadens höchstens 0,5 µm bis 5 µm radial vorstehen. Durch diese sehr feine Rippenausbildung wird weiterhin eine relativ glatte Oberfläche der Monofilamentfäden gewährleistet, während gleichzeitig eine hinreichende Stabilität an den Kreuzungspunkten der Monofilamentfäden im Gewebe und damit eine stabile Formgebung der Poren gegeben ist. Zusätzlich kann zur üblichen Veredelung, die eine Thermofixierung umfasst, das Monofilamentgewebe kalandriert sein.

Eine weitere bevorzugte Ausführungsform der Erfindung besteht darin, dass die Auflageseite zumindest bereichsweise mit einer Haftschicht versehen ist. Die Haftschicht ist dabei üblicherweise ein Klebstoffauftrag, welcher zum Aufbringen auf Haut geeignet ist. Die Haftschicht kann dabei unmittelbar auf der Auflageseite des Monofilamentgewebes aufgebracht werden.

Diese Ausführung der Erfindung ist in bevorzugter Weise dadurch weitergebildet, dass die Haftschicht streifenförmig entlang eines Randbereiches der Auflageseite des Monofilamentgewebes aufgebracht ist. In einer Weiterbildung kann die von der Auflageseite abgewandte Oberseite des Randbereiches des Monofilamentgewebes von dem Deckelement freigehalten sein.

Bei dieser Ausführungsform erstreckt sich also das Deckelement nicht über die gesamte Oberfläche des Monofilamentgewebes, sondern lediglich in einem Mittenbereich, während ein streifenförmiger Randbereich freigehalten ist. Der Randbereich kann sich nur entlang eines Teilbereiches des Außenumfanges erstrecken oder entlang des gesamten Außenumfanges ausgebildet und damit ringförmig geschlossen sein. Durch diese streifenförmige Haftschicht wird eine Fixierung des Wundauflagemateriales beabstandet zur Wunde ermöglicht. Durch ein Freihalten der Oberseite des Randbereiches wird ein hinreichender Luftzutritt ermöglicht und einer Irritation der Haut durch die Haftschicht entgegengewirkt.

Der Randbereich des Wundauflagemateriales kann besäumt sein, insbesondere durch eine Ultraschallbesäumung oder eine thermische Besäumung (Heißschnitt), bei welcher die Schnittkanten verschweißt und Schneidkanten abgerundet werden. Dabei kann die Haftschicht auch auf einem Auflageelement aufgebracht sein, welches wiederum entlang des Randbereiches des Monofilamentgewebes aufgebracht ist. Dieses Auflageelement kann dabei um die Randkante des Monofilamentgewebes umgeschlagen sein und dieses zusätzlich umsäumen, um so einen Tragekomfort weiter zu erhöhen.

Gemäß einer Weiterbildung der Erfindung ist es vorteilhaft, dass das Deckelement ein Gewebe, ein non-woven Textil, eine Folie, eine Membran und/oder einen Schaum aufweist. Das Deckelement kann insbesondere selbst einen schichtförmigen oder mehrlagigen Aufbau aufweisen, wobei die einzelnen Schichten oder Lagen selbst aus gleichen oder unterschiedlichen Materialien gebildet sind. Bei der Verwendung eines Gewebes kann insbesondere neben einem weiteren Monofilamentgewebe auch ein Multifilamentgewebe mit einer erhöhten Saugfähigkeit Anwendung finden. Zu diesem Zweck können auch non-woven Textilien, also etwa ein Gewirk, ein Vlies, ein Filz oder ähnliches oder auch Watte zum Einsatz kommen. Diese Materialien sind auch gut geeignet, pflegende oder medizinisch wirksame Substanzen aufzunehmen. Für einen Schutz gegen den Zutritt von Flüssigkeiten, Gasen oder weiteren physikalischen Einwirkungen von außen kann eine Folie, eine Membran und/oder ein Schaum vorgesehen sein. Die Membran kann dabei vorzugsweise semipermeabel sein, so dass diese zwar einen Gasaustausch zulässt, jedoch keinen Eintritt von Flüssigkeit von außen in die Wunde oder umgekehrt von Flüssigkeit aus der Wunde in einen Außenbereich.

Nach der Erfindung ist es vorgesehen, dass das Deckelement mit dem Monofilamentgewebe punktverschweißt ist. Dies kann über ein thermisches Verschweißen oder vorzugsweise über ein Ultraschallverschweißen erfolgen. Das Verschweißen kann auch bei einem Heißzuschnitt oder Laserschnitt im Randbereich erfolgen. Das Deckelement kann entlang des Randbereiches linienförmig mit dem Monofilamentgewebe verschweißt sein, so dass das Deckelement an seiner Außenseite insbesondere umfassend oder ringförmig geschlossen ist.

Eine weitere vorteilhafte Ausführungsform der Erfindung besteht darin, dass das Deckelement eine transparente äußere Deckschicht aufweist. Diese kann mit dem Monofilamentgewebe unter Ausbildung mindestens eines Aufnahmebereiches verbunden sein. Die transparente Deckschicht schützt die Wandauflage nach außen. Je nach Anwendung kann die Deckschicht durchlässig oder sperrend für Feuchtigkeit und/oder Gas sein. Der mindestens eine Aufnahmebereich kann dabei ein Hohlraum sein, in welchem ein weiteres Element zur Aufnahme von Flüssigkeit oder einer Substanz vorgesehen ist. Die äußere Deckschicht kann dabei insbesondere eine Folie oder eine Membran sein, welche einen flüssigkeitsdichten Abschluss nach außen bildet. Hierdurch kann insbesondere erreicht werden, dass auf dem Wundauflagematerial getragene Bekleidungsstücke nicht durch Substanzen aus dem Wundauflagematerial oder durch Wundsekrete verschmutzt werden.

Zur Reduzierung der Gewebeirritation ist es nach einer weiteren Ausführungsvariante der Erfindung vorgesehen, dass die Dicke der durch das Deckelement gebildeten Oberseite 60% bis 98% der Gesamtdicke des Wundauflagemateriales beträgt. Das biokompatible Monofilamentgewebe ist dabei sehr fein und weist eine Dicke von 40 µm bis 1 mm, vorzugsweise zwischen 70 µm bis 300 µm auf. Das Deckelement, welches die Oberseite des Wundauflagemateriales bildet, kann abhängig vom Anwendungsfall zwischen 60 µm bis zu einigen Millimetern betragen.

Zur Förderung der Wundheilung ist es gemäß einer weiteren erfindungsgemäßen Ausführungsform vorgesehen, dass mehrere Deckelemente vorgesehen sind, wobei zumindest ein weiteres Deckelement eine pflegende oder medizinisch wirksame Substanz aufweist oder zum Aufnehmen von Wundsekret ausgebildet ist. Das Deckelement kann dabei ein Reservoir mit einer Flüssigkeits- oder Gelfüllung oder zur Aufnahme von Pulvern oder Feststoffen ausgebildet sein.

Grundsätzlich ist es vorgesehen, dass die Monofilamentfäden aus einem Kunststoffmaterial mit einer hohen Reinheit und so gefertigt werden, dass diese von alleine eine möglichst glatte Oberfläche aufweisen. Eine vorteilhafte Ausführungsform der Erfindung besteht darin, dass das Monofilamentgewebe oder die Monofilamentfäden, welche das Monofilamentgewebe bilden, mit einer Beschichtung versehen sind. Mit dieser Beschichtung kann die Oberfläche in einer gewünschten Weise behandelt und beeinflusst werden. Insbesondere sind eine hydrophile oder eine hydrophobe Beschichtung möglich. Zudem kann die Rauigkeit und die Anhafteigenschaft der Oberfläche weiter reduziert werden. Neben einer derartigen Oberflächenbeschichtung mit geeigneten Materialien kann zudem auch eine andere Oberflächenmodifikation, insbesondere eine Glättung der Fadenoberflächen oder der Oberfläche des fertigen Monofilamentgewebes eingestellt werden.

Eine weitere vorteilhafte Ausführung der Erfindung kann darin gesehen werden, dass das Monofilamentgewebe mit einer gleichmäßigen definierten Porengröße gewebt ist, wobei die Porengrößen nicht mehr als 10%, vorzugsweise weniger als 5% voneinander abweichen. Es wird also ein Monofilamentgewebe geschaffen, welches über die gesamte Fläche eine sehr gleichmäßige Porengröße aufweist. Abhängig vom Einsatzzweck kann so eine möglichst zweckmäßige Porengröße vorgesehen werden, welche einerseits etwa einen Luft- oder Flüssigkeitsdurchtritt in gewünschter Weise ermöglicht, während andererseits die Gefahr eines Einwachsens oder Umgreifens von Gewebematerial weitgehend entgegengewirkt ist.

Eine weitere bevorzugte Ausführungsform der Erfindung besteht darin, dass mindestens ein Deckelement mit dem Markermaterial bedruckt oder beschichtet ist. Das Aufbringen kann dabei auf einer oder auf beiden Seiten erfolgen. Das Bedrucken oder Beschichten kann dabei ausschließlich als eine Oberflächenbehandlung erfolgen, so dass ein Verbrauch an Markermaterial begrenzt ist. Das Bedrucken oder Beschichten ist insbesondere vorteilhaft, wenn das Deckelement aus einem Bahnmaterial hergestellt wird.

Grundsätzlich kann als ein Markermaterial, welches als ein Indikator zum Anzeigen des Wundzustands, insbesondere eines mikrobiologischen Wundmilieus und insbesondere einer Verkeimung dient, jedes geeignete Markermaterial verwendet werden. Besonders bevorzugt ist es nach einer Weiterbildung der Erfindung, dass das Markermaterial zumindest einen Farbstoff, insbesondere einen reaktiven Farbstoff, insbesondere einen reaktiven Farbstoff, einen fluoreszierenden Stoff, einen pH-empfindlichen Stoff, Enzyme, Zellen, ein Hydrogel, einen temperaturempfindlichen Stoff, ein Salz, eine Salbe und/oder Alginate umfasst.

Nach der Erfindung ist ein Verfahren zum Herstellen einer Wundauflage mit einem Markermaterial, insbesondere wie es zuvor beschrieben worden ist, vorgesehen, bei dem ein biokompatibles Monofilamentgewebe als ein erstes Bahnmaterial gebildet wird, ein Deckelement als ein zweites Bahnmaterial gebildet werden kann, welches mit dem Markermaterial versehen wird, das erste Bahnmaterial und das zweite Bahnmaterial zusammengeführt werden, wobei das biokompatible Monofilamentgewebe und das Deckelement fest miteinander verbunden, insbesondere verschweißt werden, und das Wundauflagematerial als ein Band hergestellt wird. Das so hergestellte Band kann zu einer Rolle aufgespult oder in gewünschter Weise beschnitten, konfektioniert und mit dem Markermaterial bedruckt oder beschichtet werden.

Das erfindungsgemäße Verfahren ermöglicht eine effiziente Herstellung der Wundauflage in einem kontinuierlichen Verfahren. Die Ausgangsmaterialien werden dabei vorzugsweise von Rollen als bandförmige Bahnen zugeführt. Das Deckelement kann dabei ein vorgefertigtes Bahnmaterial sein, welches aus mehreren Lagen besteht. Es ist aber ebenso möglich, dass das Deckelement aus mehreren Bahnmaterialien gebildet wird, welche gleichzeitig zugeführt und mit dem Monofilamentgewebe verbunden werden.

## Patentansprüche

1. Wundauflage mit einem Markermaterial zum Anzeigen eines Zustands einer Wunde, insbesondere eines mikrobiologischen Wundmilieus,
**dadurch gekennzeichnet,**
- **dass** die Wundauflage eine Auflageseite aufweist, welche durch ein biokompatibles Monofilamentgewebe aus Monofilamentfäden mit einer glatten Oberfläche gebildet ist, und
- **dass** auf dem Monofilamentgewebe an seiner von der Wunde abgewandten Oberseite mindestens ein Deckelement angebracht ist, welches mit dem Markermaterial versehen ist.

2. Wundauflage nach Anspruch 1,
**dadurch gekennzeichnet,**
**dass** das Monofilamentgewebe als ein Einfachgewebe mit offenmaschiger Struktur gebildet ist, welches eine Porengröße von 5 µm bis 500 µm, vorzugsweise 20 µm bis 300 µm, aufweist, und
**dass** die Poren einen Anteil von 15% bis 70% der Fläche des Monofilamentgewebes bilden.

3. Wundauflage nach Anspruch 1 oder 2,
**dadurch gekennzeichnet,**
**dass** die Monofilamentfäden des Monofilamentgewebes aus PA, PET, PP, PVDF und/oder PTFE gebildet sind und eine Durchmessergröße zwischen 20 µm bis 500 µm, vorzugsweise zwischen 30 µm bis 150 µm, aufweisen.

4. Wundauflage nach einem der Ansprüche 1 bis 3,
**dadurch gekennzeichnet,**
**dass** die Auflageseite zumindest bereichsweise mit einer Haftschicht versehen ist.

5. Wundauflage nach Anspruch 4,
**dadurch gekennzeichnet,**
**dass** die Haftschicht streifenförmig entlang eines Randbereiches der Auflageseite des Monofilamentgewebes aufgebracht ist.

6. Wundauflage nach einem der Ansprüche 1 bis 5,
**dadurch gekennzeichnet,**
**dass** das Deckelement ein Gewebe, ein non-woven Textil, eine Folie, eine Membran und/oder einen Schaum aufweist.

7. Wundauflage nach einem der Ansprüche 1 bis 6,
**dadurch gekennzeichnet,**
**dass** das Deckelement mit dem Monofilamentgewebe punktverschweißt ist, insbesondere durch Ultraschallverschweißen.

8. Wundauflage nach einem der Ansprüche 1 bis 7,
**dadurch gekennzeichnet,**
**dass** das Deckelement eine transparente äußere Schutzschicht aufweist.

9. Wundauflage nach einem der Ansprüche 1 bis 8,
**dadurch gekennzeichnet,**
**dass** die Dicke der durch das Deckelement gebildeten Oberseite 60% bis 98% der Gesamtdicke des Wundauflagemateriales beträgt.

10. Wundauflage nach einem der Ansprüche 1 bis 9,
**dadurch gekennzeichnet,**
**dass** mehrere Deckelemente vorgesehen sind, wobei zumindest ein weiteres Deckelement eine pflegende oder medizinisch wirksame Substanz aufweist oder zum Aufnehmen von Wundsekret ausgebildet ist.

11. Wundauflage nach einem der Ansprüche 1 bis 10,
**dadurch gekennzeichnet,**
**dass** das Monofilamentgewebe oder die Monofilamentfäden, welche das Monofilamentgewebe bilden, mit einer Beschichtung versehen sind.

12. Wundauflage nach einem der Ansprüche 1 bis 11,
**dadurch gekennzeichnet,**
**dass** das Monofilamentgewebe mit einer gleichmäßigen definierten Porengröße gewebt ist, wobei die Porengrößen nicht mehr als 10% voneinander abweichen.

13. Wundauflage nach einem der Ansprüche 1 bis 12,
**dadurch gekennzeichnet,**
**dass** das mindestens eine Deckelement mit dem Markermaterial bedruckt oder beschichtet ist.

14. Wundauflage nach einem der Ansprüche 1 bis 13,
**dadurch gekennzeichnet,**
**dass** das Markermaterial zumindest einen Farbstoff, insbesondere einen reaktiven Farbstoff, einen fluoreszierenden Stoff, einen pH-empfindlichen Stoff, Enzyme, Zellen, ein Hydrogel, einen temperaturempfindlichen Stoff, ein Salz, eine Salbe und/oder Alginate umfasst.

15. Verfahren zum Herstellen einer Wundauflage mit einem Markermaterial nach einem der Ansprüche 1 bis 14, bei dem
- ein biokompatibles Monofilamentgewebe als ein erstes Bahnmaterial gebildet wird,
- ein Deckelement als ein zweites Bahnmaterial gebildet wird, und
- das erste Bahnmaterial und das zweite Bahnmaterial zusammengeführt werden, wobei das biokompatible Monofilamentgewebe und das Deckelement durch Punktverschweißen fest miteinander verbunden werden,
- wobei das Deckelement mit dem Markermaterial versehen wird.

16. Verfahren nach Anspruch 15,
**dadurch gekennzeichnet,**
**dass** das Markermaterial vor und/oder nach dem Zusammenführen der beiden Bahnmaterialien auf das Deckelement aufgebracht, insbesondere aufgedruckt oder beschichtet wird.

## Claims

1. Wound dressing with a marker material for indicating a condition of a wound, in particular a microbiological wound environment,
**characterized in that**
- the wound dressing has a contact side which is formed by a biocompatible monofilament fabric of monofilament threads with a smooth surface and
- **in that** on the monofilament fabric on its upper side facing away from the wound at least one cover element is fixed which is provided with the marker material.

2. Wound dressing according to claim 1,
**characterized in that**
the monofilament fabric is formed as a single-weave fabric with an open-mesh structure having a pore size ranging from 5 µm to 500 µm, preferably from 20 µm to 300 µm, and
**in that** the pores constitute a proportion of 15 % to 70 % of the surface of the monofilament fabric.

3. Wound dressing according to claim 1 or 2,
**characterized in that**
the monofilament threads of the monofilament fabric are formed of PA, PET, PP, PVDF and/or PTFE and have a diameter size ranging between 20 µm and 500 µm, preferably between 50 µm and 150 µm.

4. Wound dressing according to any one of claims 1 to 3,
**characterized in that**
the contact side is provided at least in some areas with an adhesive layer.

5. Wound dressing according to claim 4,
**characterized in that**
the adhesive layer is applied in a strip-shaped manner along a marginal area of the contact side of the monofilament fabric.

6. Wound dressing according to any one of claims 1 to 5,
**characterized in that**
the cover element has a fabric, a non-woven textile, a film, a membrane and/or a foam.

7. Wound dressing according to any one of claims 1 to 6,
**characterized in that**
the cover element is spot-welded to the monofilament fabric, in particular through ultrasonic welding.

8. Wound dressing according to any one of claims 1 to 7,
**characterized in that**
the cover element has a transparent external protective layer.

9. Wound dressing according to any one of claims 1 to 8,
**characterized in that**
the thickness of the upper side formed by the cover element amounts to 60 % to 98 % of the overall thickness of the wound dressing material.

10. Wound dressing according to any one of claims 1 to 9,
**characterized in that**
several cover elements are provided, wherein at least one further cover element has a care substance or medically effective substance or is designed to receive wound secretions.

11. Wound dressing according to any one of claims 1 to 10,
**characterized in that**
the monofilament fabric or the monofilament threads that form the monofilament fabric are provided with a coating.

12. Wound dressing according to any one of claims 1 to 11,
**characterized in that**
the monofilament fabric is woven with a uniform defined pore size, wherein the pore sizes do not differ from each other by more than 10%.

13. Wound dressing according to any one of claims 1 to 12,
**characterized in that**
the at least one cover element is imprinted or coated with the marker material.

14. Wound dressing according to any one of claims 1 to 13,
**characterized in that**
the marker material comprises at least one colorant, in particular a reactive colorant, a fluorescent substance, a pH-sensitive substance, enzymes, cells, a hydrogel, a temperature-sensitive substance, a salt, an ointment and/or alginates.

15. Method for producing a wound dressing with a marker material according to any one of claims 1 to 14, in which
- a biocompatible monofilament fabric is formed as a first web material,
- a cover element is formed as a second web material, and
- the first web material and the second web material are joined, wherein the biocompatible monofilament fabric and the cover element are firmly connected to each other through spot welding,
- wherein the cover element is provided with the marker material.

16. Method according to claim 15,
**characterized in that**
the marker material is applied to the cover element, in particular imprinted or coated thereon, before and/or after joining of the two web materials.

## Revendications

1. Pansement comportant un matériau de marquage pour indiquer un état d'une plaie, en particulier d'un milieu microbiologique de la plaie,
**caractérisé en ce**
- **que** le pansement présente un côté d'application qui est formé par un tissu monofilament biocompatible en fils monofilaments comportant une surface lisse, et
- **que** sur le tissu monofilament sur son côté supérieur opposé à la plaie est placé au moins un élément de recouvrement qui est doté du matériau de marquage.

2. Pansement selon la revendication 1,
**caractérisé en ce**
**que** le tissu monofilament est formé comme un tissu simple comportant une structure à mailles ouvertes qui présente une taille de pore de 5 µm à 500 µm, de préférence de 20 µm à 300 µm, et
**que** les pores forment une proportion de 15 % à 70 % de la surface du tissu monofilament.

3. Pansement selon la revendication 1 ou 2,
**caractérisé en ce**
**que** les fils monofilaments du tissu monofilament sont formés en PA, PET, PP, PVDF et/ou PTFE et présentent un diamètre entre 20 µm et 500 µm, de préférence entre 30 µm et 150 µm.

4. Pansement selon l'une des revendications 1 à 3,
**caractérisé en ce**
**que** le côté d'application est doté au moins par endroits d'une couche adhésive.

5. Pansement selon la revendication 4,
**caractérisé en ce**
**que** la couche adhésive est appliquée en forme de bande le long d'une zone de bordure du côté d'application du tissu monofilament.

6. Pansement selon l'une des revendications 1 à 5,
**caractérisé en ce**
**que** l'élément de recouvrement présente un tissu, un textile non tissé, une feuille, une membrane et/ou une mousse.

7. Pansement selon l'une des revendications 1 à 6,
**caractérisé en ce**
**que** l'élément de recouvrement est soudé par points avec le tissu monofilament, en particulier par soudage à ultrasons.

8. Pansement selon l'une des revendications 1 à 7,
**caractérisé en ce**
**que** l'élément de recouvrement présente une couche de protection externe transparente.

9. Pansement selon l'une des revendications 1 à 8,
**caractérisé en ce**
**que** l'épaisseur du côté supérieur formé par l'élément de recouvrement est de 60 % à 98 % de l'épaisseur totale du matériau du pansement.

10. Pansement selon l'une des revendications 1 à 9,
**caractérisé en ce**
**que** plusieurs éléments de recouvrement sont prévus, au moins un autre élément de recouvrement présentant une substance traitante ou médicalement active ou est conçu pour recevoir des sécrétions de la plaie.

11. Pansement selon l'une des revendications 1 à 10,
**caractérisé en ce**
**que** le tissu monofilament ou les fils monofilaments qui forment le tissu monofilament sont dotés d'un revêtement.

12. Pansement selon l'une des revendications 1 à 11,
**caractérisé en ce**
**que** le tissu monofilament est tissé en comportant une taille de pore définie régulière, dans lequel les tailles de pores ne diffèrent pas de plus de 10 % les unes des autres.

13. Pansement selon l'une des revendications 1 à 12,
**caractérisé en ce**
**que** l'au moins un élément de recouvrement est imprimé ou revêtu d'un matériau de marquage.

14. Pansement selon l'une des revendications 1 à 13,
**caractérisé en ce**
**que** le matériau de marquage comprend au moins un colorant, en particulier un colorant réactif, une substance fluorescente, une substance sensible au pH, des enzymes, des cellules, un hydrogel, une substance sensible à la température, un sel, une crème et/ou des alginates.

15. Procédé de fabrication d'un pansement comportant un matériau de marquage selon l'une des revendications 1 à 14, dans lequel
- un tissu monofilament biocompatible est formé en tant que premier matériau en bande,
- un élément de recouvrement est formé en tant que deuxième matériau en bande, et
- le premier matériau en bande et le deuxième matériau en bande sont réunis, le tissu monofilament biocompatible et l'élément de recouvrement étant reliés fixement l'un à l'autre par un soudage par points,
- l'élément de recouvrement étant doté du matériau de marquage.

16. Procédé selon la revendication 15,
**caractérisé en ce**
**que** le matériau de marquage est appliqué avant et/ou après la réunion des deux matériaux en bande sur l'élément de recouvrement, en particulier, est imprimé ou revêtu.
